(19) Europäisches Patentamt
European Patent Office
Office européen des brevets

(11) **EP 0 768 870 B1**

(12) **EUROPEAN PATENT SPECIFICATION**

(45) Date of publication and mention
of the grant of the patent:
**08.11.2000 Bulletin 2000/45**

(51) Int. Cl.[7]: **A61K 9/16, A61K 9/50**

(86) International application number:
**PCT/EP95/02468**

(21) Application number: **95924932.7**

(22) Date of filing: **24.06.1995**

(87) International publication number:
**WO 96/01103 (18.01.1996 Gazette 1996/04)**

(54) **MULTIPLE ENCAPSULATION OF OLEOPHILIC SUBSTANCES**

MEHRFACHE VERKAPSELUNG VON ÖLLÖSLICHE VERBINDUNGEN

ENCAPSULATION MULTIPLE DE SUBSTANCES OLEOPHILES

(84) Designated Contracting States:
**AT BE CH DE DK ES FR GB IT LI NL**

(30) Priority: **01.07.1994 US 269720**
**24.05.1995 US 439787**

(43) Date of publication of application:
**23.04.1997 Bulletin 1997/17**

(73) Proprietor:
**F. HOFFMANN-LA ROCHE AG**
**4002 Basel (CH)**

(72) Inventors:
• **BOYLE, Stasia**
**Wayne, NJ 07470 (US)**

• **CHANG, Kuei-Tu**
**Mountain Lakes, NJ 07046 (US)**

(74) Representative:
**Kellenberger, Marcus, Dr.**
**F.Hoffmann-La Roche AG**
**Patent Department (PLP),**
**124 Grenzacherstrasse**
**4070 Basel (CH)**

(56) References cited:
**EP-A- 0 462 003          EP-A- 0 595 030**
**WO-A-88/08300          WO-A-89/07935**

**Description**

The present invention relates to a process for the encapsulation of oleophilic substances and to compositions produced by this process. In particular, the invention relates to the preparation of high potency, dry and free-flowing vitamin powders or beadlets using multiple microspheric and/or microencapsulation techniques.

Oleophilic substances (fat- and oil-soluble substances, as well as fats and oils), and in particular fat-soluble vitamins such as vitamins A, D, E and K, have in the past been formulated with difficulty into dry, free-flowing materials because of their oil-like nature. Thus, oleophilic substances have tended to be macroencapsulated and delivered as units of coated oil. Unfortunately, however, the nature of such formulations drastically restricts the applications for which the oleophilic substances are suitable. For example, current commercial techniques can only produce vitamin E formulations with concentrations of vitamin E which range up to 50% by weight.

The present invention overcomes such limitations inherent in the prior art encapsulation techniques and enables the production of high potency vitamin compositions in a free-flowing powder form. Such forms are suitable for incorporation into multiple vitamin tablets or foods, such as cereals, and provide the advantages of reducing tablet size or the bulk required for subsequent delivery. Of particular interest in this respect are high potency vitamin E formulations, as dry powders or beadlets, in which the vitamin E content is greater than about 50% by weight.

The prior art, as represented for example by Lim et al. in U.S. Patent No. 4,389,419, describes the formation of an emulsion consisting of a continuous phase aqueous solution of an alkali metal alginate and a dispersed phase of an oleophilic substance, and optionally, a water-soluble, alcohol-insoluble filler such as a polysaccharide or methyl cellulose. The emulsion thus produced is then formed into droplets which are emersed into an alcoholic solution of multivalent cations to produce a water-insoluble, shape-retaining alginate matrix, preferably filled with precipitated polysaccharide, which encloses a plurality of oil droplets.

The Lim et al. approach differs markedly from that of the present invention in which an oleophilic substance is incorporated into a primary polymer-containing solution which is then solidified under mixing conditions to encapsulate the fat-soluble substance and thereby form an appropriate composition. Through the use of mixing conditions to form the primary particles, far greater concentrations of oleophilic substances relative to those achieved by Lim et al. can be realized. As stated by Lim et al., the amount of oil (particularly fat- or oil-soluble vitamin) may range from 1% up to nearly 30%. However, at the higher end of this range, the stability of the Lim et al. oil-in-water emulsion is decreased and the quality of the Lim et al. microcapsules is reduced. Additionally, the Lim et al. droplets must be removed from the alcoholic solution and washed, or otherwise treated, to remove any residual alcohol, thus requiring extra production steps.

Another markedly different approach employed for encapsulating vitamin or mineral nutrients, such as thiamine, is described by Hall et al. in U.S. Patent No. 4,182,778. Hall et al. describes encapsulation by fluidizing the nutrient in a gaseous stream and contacting the nutrient with finely atomized droplets of a coating solution. Nowhere, however, is the Hall et al. encapsulation process suggested to be applicable to oleophilic substances.

As described below, the concentration of the oleophilic substance in the compositions of the present invention typically ranges from about 30% to about 90% based on the dry weight of the final encapsulated oleophilic composition. These percentages are far superior to those described by Lim et al., and form a major breakthrough over the current state of the art which only allows percentages of vitamin E to reach about 50%. Thus, the present invention fulfils a long-felt need in the art for high potency encapsulated oleophilic compositions.

The present invention provides a method for producing a composition of an oleophilic substance encapsulated by a polymer, which method is characterized by (a) incorporating an oleophilic substance into a primary polymer-containing solution and (b) solidifying the primary polymer under mixing conditions to encapsulate the oleophilic substance in the polymer and thereby form the composition of the oleophilic substance encapsulated by the primary polymer, and optionally (c) mixing the so-produced primary polymer-encapsulated olephilic substance as particles with a further (secondary) polymer to encapsulate the primary polymer-encapsulated oleophilic substance particles in the secondary polymer and thereby form the composition of the oleophilic substance encapsulated by primary and secondary polymers. The mixing conditions may vary between the first and the second encapsulation.

Thus one aspect of the present invention is the method as above comprising the process steps (a) and (b) [but not (c)], and a further aspect is the method comprising all three process steps (a), (b) and (c). The product of steps (a) and (b) is generally in the form of particles, which for convenience will be referred to hereinafter as primary particles. Furthermore, the product of steps (a), (b) and (c) is also generally in the form of particles, hereinafter referred to for convenience as secondary particles. Since the oleophilic substance involved may consist of more than one separate kind, the primary particles will in these circumstances consist of the appropriate different kinds (hereinafter "multiple primary particles"), viz, one kind of oleophilic substance encapsulated by the primary polymer and more than one kind (at least two kinds) of oleophilic substance encapsulated by the primary polymer. If process step (c) is utilized, and more than one kind of oleophilic substance has been incorporated into the primary polymer-containing solution in process step (a), then the multiple primary particles may be encapsulated by the secondary polymer to afford secondary particles including in each case one or more kinds of oleophilic substance.

Process step (c) of the present invention may be followed by an analogous (or repeated) process step [(d)] involving the same kind of or a different polymer as/than that (the secondary polymer) used in process step (c). This (same or different) polymer is conveniently referred to hereinafter as the tertiary polymer. Through the use of process step (d) the oleophilic substance (one or more kinds) is ultimately encapsulated in turn by three polymers, viz, directly by the primary polymer, beyond this by the secondary polymer, and finally by the tertiary polymer, whereby the primary and secondary particles may be encapsulated in each case individually or in groups of two or more. The employment of said process step (d) represents a further aspect of the present invention.

The present invention further provides the product obtainable by or obtained by the above defined method in all its aspects, as a composition in the appropriate form indicated above, viz, as primary particles, as multiple primary particles, as secondary particles including one or more kinds of oleophilic substance, and as secondary particles encapsulated individually or collectively (in groups) by a tertiary polymer and also in particulate form. Under the term "particles" or "particulate" is especially to be understood in this context powder/pulverous or beadlet(s), as appropriate. Preferably the concentration of the oleophilic substance in the product ranges from about 30% to about 90% based on the dry weight of the final composition.

Throughout this specification the term "oleophilic substance" refers to a fat- or oil-soluble substance, a fat or an oil, and especially to the (fat-soluble) vitamin A, D, E or K, whereby derivatives of such vitamins are also contemplated, such as vitamin A acetate, vitamin A palmitate and vitamin E acetate.

The primary polymer is preferably a cellulose, such as methylcellulose or hydroxypropyl methylcellulose, whereas the secondary and tertiary polymer is generally a cellulose, e.g. methylcellulose or hydroxypropyl methylcellulose, a cellulose derivative, especially hydroxypropyl methylcellulose phthalate, a maltodextrin, such as a maltodextrin having a dextrose equivalent value (D.E.) of about 18, an alginic acid derivative (alginate), e.g. sodium, potassium or propylene glycol alginate, calcium lactate, gum acacia, a gelatin, such as fish gelatin, or modified starch, such as hydroxypropyl starch or pregelatinized corn starch.

Preferred celluloses include methylcellulose and hydroxypropyl methylcellulose, and preferred cellulose derivatives include hydroxypropyl methylcellulose phthalate.

The first process step [(a)] in the method of the present invention involves incorporating the oleophilic substance into the primary polymer-containing solution, typically an aqueous solution. Said "incorporating" embraces emulsifying, dispersing to form a suspension, dissolving or diluting, whereby more than one of such techniques may be involved in a single step (a). Thus, for example, the step (a) may be effected by (1) emulsifying a vitamin, e.g. vitamin A, D, E or K or a derivative thereof, in an aqueous solution of the (primary) polymer, such as a cellulose or a cellulose derivative, or (2) dispersing a vitamin in the form of crystals in an aqueous solution of the primary polymer to form a suspension, or (3) dissolving and/or diluting a fat-soluble vitamin in an oil and then emulsifying the vitamin-oil solution in an aqueous solution of the primary polymer to form an emulsion.

In general, the temperature at which the oleophilic substance is incorporated in the primary polymer-containing solution to form an emulsion or suspension is not critical. A practical temperature range for this step depends mainly on the nature of the polymer chosen and the concentration of the polymer-containing solution, and is not so high as to cause premature solidification (polymerization; polymeric setting). For example, in preparing an emulsion with hydroxypropyl methylcellulose, the temperature should preferably be between room temperature and 60°C. The person skilled in the art is readily able to determine an acceptable temperature range based on the components chosen.

Then, after the incorporation (emulsifying, dispersing, dissolving and/or diluting), the emulsion or suspension is typically either heated to a polymeric setting temperature (typically greater than 50°C) under mixing conditions to produce primary particles (e.g. microcapsules and/or microspheres), or the polymer can be cross-linked, to effect the process step (b). In each case the oleophilic substance is present as a liquid during at least a part of the processing time, and typically throughout processing. The solidification is generally effected by heating (thermal setting), cross-linking or coacervating.

The term "mixing conditions" used in step (b) refers to a state of imparted movement, such as that associated with stirring, shaking, agitating, jumbling etc., which is vigorous enough to cause the emulsion or suspension to solidify as particles. Mixing conditions are readily determined by a person skilled in the art using known techniques, for example, by using an anchor stirrer, a homogenizer, a colloidal mill, a microfluidizer, a sonicator, or an impeller (mechanical stirring). Particle size can be varied by changing the amount of imparted movement. For example, slow stirring will generally produce a larger particle size than will fast stirring. The Examples which follow provide ample guidance for one skilled in the art to determine appropriate mixing conditions without undue experimentation.

The solidification step (b) for making the primary particles is physically determined by the mechanism and process temperature used. For instance, if solidification is caused by cross-linking, such as with an alginate emulsion cross-linking with polyvalent cations, e.g. calcium, zinc, aluminium, copper or silver, the temperature may not be critical, i.e. processing can be effected at any reasonable temperature. However, when methylcellulose or a derivative thereof, or hydroxypropyl methylcellulose or a derivative thereof, is chosen as the primary polymer and heating is the mechanism utilized for the solidification, temperature becomes important and must be maintained above about 50°C (for a 2% aqueous solution, for example, in the range about 50-55°C or 50-85°C, respectively) throughout the whole process. Again, these parameters are readily determinable by the person skilled in the art.

Microcapsules are formed after the performance of process steps (a) and (b) by a single encapsulation of the oleophilic substance with the primary polymer, whereas microspheres are conglomerates of multi-oil droplets embedded in a spherical or sphere-like matrix. Typically, microspheres are formed from numerous contacting microcapsules embedded in an outer layer of polymer. The term "primary particle" as used herein refers in particular to micro- and/or macro-particles [designation according to their relative size (diameter), the border-line being about 0.2 μm, for example] produced by either heating (thermal setting), cross-linking or coacervating the polymer-containing emulsion.

Primary particles may then be further encapsulated with a secondary and optionally thereafter also with a tertiary polymeric layer (depending on whether the primary particles are microcapsules or microspheres) by the addition of a secondary polymer to the suspension resulting from the solidification of the primary particles of oleophilic substance encapsulated by polymer within the polymer-containing solution followed by solidifying the secondary polymer to form secondary particles, and optionally repeating this procedure with a tertiary polymer to afford tertiary particles. The term "secondary particle" as used herein refers to a primary particle which has been further encapsulated or coated (the term "encapsulated" also includes the term "coated"). Of course, individual or multiple primary particles (at least two) may be encapsulated or coated to form a single secondary particle, and analogous considerations apply for the formation of the tertiary particle. Such encapsulation may be achieved by changing the pH, by adding a cross-linking agent, by heating, by spray drying or by other suitable means known per se. Examples of cross-linking agents include sugars, e.g. reducing sugars, gum acacia, polyvalent cations as indicated above, e.g. calcium such as from calcium chloride, and gelatin.

The application of the further (optional) encapsulation step (c) represents a preferred feature of the method of the present invention.

Although the method of the present method may involve in step (b) solidifying the primary polymer by heating (to form the primary particles), and in step (c) coating or chemically cross-linking the secondary polymer (to form the secondary particles), alternative methods of achieving the respective encapsulations may be utilized and are embraced by the method of the present invention. Additionally, further encapsulations of the secondary particles, as invisaged by step (d) of said method, such as coating or polymerization around the secondary particles, may be effected to afford tertiary particles. The choice of suitable temperatures, pH ranges and ionic strengths for adding the secondary or tertiary polymer depends on the types of materials and mechanisms used. In respect of polymerization by adjustment of the pH, there may be mentioned as exemplification the choice of a pH below about 5 or in the range about 2-3 when hydroxypropyl methylcellulose phthalate or carboxymethylcellulose, respectively, is used as the polymer. When gelatin is crosslinked with gum acacia for this purpose, for example, the pH is generally adjusted to the range about 4-4.5 with cooling to about 5°C. Crosslinking of methylcellulose, hydroxypropyl methylcellulose and their derivatives may be effected using divalent acids such as tannic acid, tartaric acid or tannin, for example. Such and further means will be within the capabilities of the person skilled in the art and apply not only to the secondary and tertiary particles, but also to the primary particles, depending on the polymer used.

The final composition may be spray dried or processed by any suitable drying technique to powder or beadlet form which has high oleophilic substance, particularly vitamin A, D, E or K or a derivative thereof, potency, is dry and free-flowing, and is suitable for tableting or encapsulation within soft or hard gelatin capsules. Such powders may also be used in food, animal feed or pharmaceutical applications, and for these purposes converted to premix, suspension or emulsion form.

The invention is illustrated by the following Examples:

EXAMPLE 1

High Potency Vitamin E Acetate using Hydroxypropyl Methylcellulose as the Primary Polymer, Sodium Alginate as the Secondary Polymer, and Hydroxypropyl Methylcellulose as the Tertiary Polymer

| INGREDIENTS | FORMULATION I A % | FORMULATION I B % |
|---|---|---|
| Vitamin E acetate | 75 + 2% overage[1] | 75 + 2% overage[1] |
| Methocel® E 15LV[2] | 13.0 | 20.0 |
| Kelton LV[3]/Calcium chloride, weight ratio 1:0.5 | 2.0 | 2.0 |
| HPMC, 6 x 10$^{-3}$ Nsec/m$^2$ (6 cps)[4] | 10.0 | 3.0 |

[1] "Overage" is standard in the industry to ensure that the final product contains at least the given percentage. For this purpose a 2% overage was added.
[2] Methocel® E15LV refers to a special grade of hydroxypropyl methylcellulose manufactured by Dow Chemical Co.
[3] Kelton LV refers to the trade name for sodium alginate from the Kelco Division of Merck & Co. Inc.
[4] HPMC, 6 x 10$^{-3}$ Nsec/m$^2$ (6 cps) refers to hydroxypropyl methylcellulose 2910, such as that manufactured by SHI-ETSU Co. ("PHARMACOAT 606"). "Nsec/m$^2$", "cps" and "cP" are the viscosity units (the second and third being "centipoise").

PROCEDURE

    1. Aqueous sodium alginate solution (5% w/w) was prepared in a 2 liter (l) beaker as follows:

| Preparation of 5% sodium alginate solution | | | | |
|---|---|---|---|---|
| Time (min.) | Temperature (°C) | Stirrer speed (rpm) | Amount | Comments |
| 0 | 20 | | 1.27 l | Add water to beaker and stir |
| 5 | 20 | 1200 | 66.7 g | Add sodium alginate |
| | 25-70 | 2000 | | Heat suspension to 70°C, and maintain temperature and agitation until the solution is used |

    2. Aqueous calcium chloride solution (5% by weight) was prepared in a 1 l beaker as follows:

| Preparation of 5% calcium chloride solution | | | | |
|---|---|---|---|---|
| Time (min.) | Temperature (°C) | Stirrer | Amount | Comments |
| 0 | 22 | -- | 0.633 l | Add water to beaker |
| 1 | 22 | on | 33.3 g | Add calcium chloride |
| 2 | 22-70 | on | | Stir, heat to 70°C and maintain this temperature until used |

3. Aqueous HPMC, 6 x 10$^{-3}$ Nsec/m$^2$ (6 cps) solution (12% by weight) was prepared in a 5 l beaker as follows:

| Preparation of 12% HPMC, 6 cps, solution | | | | |
|---|---|---|---|---|
| Time (min.) | Temp. (°C) | Stirrer speed (rpm) | Amount | Comments |
| 0 | 21 | | 2.45 l | Add water to beaker and heat to 90°C |
| 32 | 90 | 1000 | 0.5 kg | Slowly add HPMC 6 x 10$^{-3}$ Nsec/m$^2$ (6 cps) powder with agitation |
| 52 | 86 | 200 | | Addition completed; remove heating source |
| 82 | 91 | 1000 | 1.22 l | Rapidly add cold water (rapid cooling) |
| | | | | Cool to room temperature while maintaining gentle agitation until use |

4. Aqueous Methocel® E15LV solution (10%) was prepared using the same procedure as described in step 3. Essentially, Methocel® E15LV was dispersed in water at 90°C, stirred and then uniformly dissolved in the water while the temperature was reduced.

5. Vitamin E acetate (an oil) was added to the Methocel® solution according to the above formulation, and the mixture was homogenized using a colloidal mill until an emulsion having the smallest possible oil droplet size was reached. Although average droplet size tends to vary, droplets of less than about 3 μm in diameter are satisfactory for most uses. Typically, average droplets range between about 0.5 and 1 μm in diameter, even though smaller diameters are generally preferred. (During emulsification, a cooling system was applied to maintain the temperature at approximately 25°C).

6. The emulsion was then heated to about 80-90°C while mixing slowly at about 200 revolutions per minute (rpm) with an anchor stirrer.

7. The warm (70°C) 5% aqueous sodium alginate solution was then added and the mixture was stirred slowly at about 200 rpm with an anchor stirrer while maintaining the temperature above 70°C.

8. The mixture was maintained at about 70°C for approximately 15 minutes, and then cross-linked by adding the 5% aqueous calcium chloride solution.

9. The 12% aqueous HPMC 6 x 10$^{-3}$ Nsec/m$^2$ (6 cps) solution at room temperature (RT) was added in small aliquots while maintaining the temperature above 70°C.

10. Water was then added to adjust the viscosity to less than about 2 Nsec/m$^2$ (2,000 cP) [in this particular instance 1 Nsec/m$^2$ (1,000 cP)] for spray drying.

11. The solution was then spray dried under conventional conditions.

A specific vitamin E acetate emulsion was prepared in a FRYMA processing unit (homogenizer, different types of mixers, and cooling and heating system manufactured by Fryma, Inc.) as follows:

| Preparation of the vitamin E acetate emulsion and suspension | | | | | |
|---|---|---|---|---|---|
| Time (min.) | Temp. (°C) | Anchor stirrer | Colloidal mill | Dissolver | Amount | Comments |
| 0 | 26 | on | -- | -- | 6.5 kg | Add 10% Methocel solution to vessel |

(continued)

| Preparation of the vitamin E acetate emulsion and suspension | | | | | | |
|---|---|---|---|---|---|---|
| Time (min.) | Temp. (°C) | Anchor stirrer | Colloidal mill | Dissolver | Amount | Comments |
| 10 | 26 | -- | -- | -- | 3.83 kg | Add vitamin E acetate to above solution |
| 18 | 27 | on | on | on | | Start emulsification |
| 23 | | on | on | on | | Sample 1: particle size of oil droplets 790 nm |
| 28 | | on | on | on | | Sample 2: particle size of oil droplets 713 nm |
| 33 | | on | on | on | | Sample 3: particle size of oil droplets 768 nm |
| 33 | | on | -- | -- | | Emulsification ended; begin heating to 85°C and maintain a steady agitation throughout the whole process |
| 110 | 85 | on | -- | -- | | Add sodium alginate solution (75°C) |
| 115 | 83 | on | -- | -- | | Mix 15 minutes |
| 130 | 82 | on | -- | -- | | Add calcium chloride solution (75°C) |
| 132 | 85 | on | -- | -- | | Add HPMC $6 \times 10^{-3}$ Nsec/m$^2$ (6 cps) solution (75°C) |
| 145 | 85 | on | -- | -- | | Being cooling |
| 165 | 82 | on | -- | -- | | Mix at room temperature |
| 195 | 22 | on | -- | -- | | Heat up to 75°C |
| 245 | 75 | on | -- | -- | | Measure viscosity: 4.3 Nsec/m$^2$ (4300 cP) (19°C) |
| 255 | 73 | on | -- | -- | 21 | Add water to adjust viscosity to 1 Nsec/m$^2$ (1000 cP) |

The above vitamin E acetate suspension was spray dried using a NIRO spray dryer (manufactured by Niro, Inc.) operated with air.

EP 0 768 870 B1

EXAMPLE 2

High Potency Vitamin E Acetate using Hydroxypropyl Methylcellulose as the Primary Polymer and Hydroxypropyl Methylcellulose as the Secondary Polymer

| INGREDIENTS | Formulation | | | | | |
|---|---|---|---|---|---|---|
| | 85% E | 80% E | 75% E | 70% E | 65% E | 50% E |
| Vitamin E acetate | 85 | 80 | 75 | 70 | 65 | 50 |
| Methocel® E15LV | 10.0 | 13.4 | 16.7 | 20 | 23.4 | 33.4 |
| HPMCP grade HP-55S* | 5.0 | 6.6 | 8.3 | 10 | 11.6 | 16.4 |
| Note: A 2% overage of vitamin E acetate was used for each formulation. | | | | | | |

\* HPMCP (grade) HP-55S refers to a special grade of hydroxypropyl methylcellulose phthalate (HPMCP). To dissolve the HPMCP, 4.5 ml of 0.5 N NaOH per gram of HPMCP were utilized. Once the HPMCP had completely dissolved in the alkaline solution, water was added to adjust the solution to a 15% solid content.

PROCEDURE

1. Aqueous HPMCP HP-55S solution (15% by weight) was prepared in a 100 l vessel as follows:

| Preparation of 15% HPMCP HP 55S solution | | | | |
|---|---|---|---|---|
| Time (min.) | Temp. (°C) | Stirrer | Amount | Comments |
| 0 | 16 | on | 14.94 l | Add water to the vessel |
| 5 | 15 | on | 300 g | Add sodium hydroxide slowly |
| 10 | 16 | on | | Addition ended |
| 15 | | on | 3.32 kg | Add HPMCP HP-55S powder to above solution with agitation |
| 35 | 14 | on | | Addition ended, start heating to 70°C |
| 215 | 65 | on | 3.57 l | Add hot water (70°C) Stir the solution until use |

2. Aqueous Methocel® E15LV solution (10%) was prepared as in Example 1.

3. Vitamin E acetate was added to the Methocel® solution according to the above formulations and the mixture homogenized to produce an emulsion as in Example 1.

4. The resulting emulsion was heated to 80-90°C with slow (200 rpm) mixing with an anchor stirrer.

5. An amount of warm (70°C) 15% aqueous HPMCP solution was added. The amount added corresponded to the amount recited in the percentage table shown above.

6. The temperature of the mixture was kept at about 80°C, and cross-linking was effected by adding 0.1 N hydrochloric acid, with mixing, to shift the pH to approximately 5 (about 4.9-5.2).

7. The cross-linked solution was allowed to equilibrate for approximately 45 minutes.

8

8. The resulting cross-linked solution was then spray dried under the conditions shown below to form a powder.

| Spray drying of the vitamin E acetate suspension | | | | | | | | |
|---|---|---|---|---|---|---|---|---|
| The suspension was pumped from the FRYMA processing unit to the spray tower by a gear pump and then atomized by a rotary atomizer from Niro, Inc. | | | | | | | | |
| Time (min.) | Set inlet temp. (°C) | Inlet temp. (°C) | Set outlet temp. (°C) | Outlet temp. (°C) | Set pressure tower in mbar | Pressure tower in mbar | Inlet air flow in m3/h | Comments |
| 0 | 180 | | 100 | | -5 | | | Start up lower* |
| 50 | 180 | 181 | 100 | 101 | -5 | -3 | 1500 | Start spray |
| 60 | 180 | 181 | 100 | 100 | -5 | -3 | 1500 | |
| 62 | | | | | | | | Spray drying ended |
| The "Set pressure tower" is the setting for the equilibrium condition in the dryer. In contrast, the "Pressure tower" is the actual pressure reached in the dryer during spray drying. | | | | | | | | |

* Indicates start up the spray dryer from a lower inlet temperature. It is standard procedure to start up a spray dryer at low temperature, then gradually increase the inlet temperature to reach a proper outlet temperature, and to establish an equilibrium condition.

EXAMPLE 3

High Potency Vitamin E Acetate using Hydroxypropyl Methylcellulose as the Primary Polymer and Fish Gelatin, Maltodextrin, Pregelatinized Corn Starch, Calcium Lactate or Hydroxypropyl Starch as the Secondary Polymer

Following the procedure in Example 2 (with HPMCP being replaced by another secondary polymer), several different secondary polymers were evaluated as follows:

| INGREDIENTS | Fish Gelatin 75%E | Maltrin 75%E | Pregelatinized Corn Starch 75%E | Calcium Lactate 75%E | Hydroxypropyl Starch 75%E |
|---|---|---|---|---|---|
| Vitamin E acetate | 75 | 75 | 75 | 75 | 75 |
| Methocel® E15LV | 16.7 | 16.7 | 16.7 | 16.7 | 16.7 |
| Fish gelatin | 8.3 | -- | -- | -- | -- |
| Maltrin M180[1] | -- | 8.3 | -- | -- | -- |
| Pregelatinized corn (PreGel) starch | -- | -- | 8.3 | -- | -- |
| Calcium lactate | -- | -- | -- | 8.3 | -- |
| Hydroxypropyl (HP) starch | -- | -- | -- | -- | 8.3 |

[1] Brand of maltodextrin (D.E. = 18).

Analysis of the five secondary polymers provided the following results using standard test procedures:

| | | Fish Gelatin | Maltrin M180 | PreGel Starch | CaLactate | HP Starch |
|---|---|---|---|---|---|---|
| Flow (Agway) | sec./100g | 27 | 23 | 28 | 17 | 39 |
| Density | g/ml | 0.34 | 0.39 | 0.33 | 0.50 | 0.34 |
| Tapped density | g/ml | 0.39 | 0.44 | 0.38 | 0.56 | 0.39 |
| Moisture | % | 1.3 | 1.0 | 2.8 | 0.8 | 1.0 |
| Color | W1E313[2] | 37.87 | 54.26 | 51.43 | 53.65 | 50.10 |
| | Y1E313[2] | 13.22 | 9.18 | 9.96 | 9.12 | 9.94 |
| Static | observed | High | Medium | Low | Medium | Very High |
| Free oil[3] | % | 2.4 | 1.6 | 2.3 | 2.9 | 2.2 |

[2]WI and YI represent white index and yellow index, respectively, and E313 that the color measurement was based on the "Standard test method for indexes of whiteness and yellowness of near-white opaque materials" issued by the American Society for Testing and Materials (ASTM); this standard is designated E313.

[3]Free oil refers to the percentage of unencapsulated vitamin E acetate, and was determined as follows:

Procedure for Measuring Free Oil on Powder Surface

Procedure for Preparing Sample Solution

1. Transfer 0.250 g of powder (vitamin E acetate 75%) to a suitable container.
2. Add 43.0 g of light mineral oil (Fisher).
3. Shake on a mechanical shaker at medium speed for 15 minutes.
4. Pass through a 0.45 μm filter.
5. Measure the u.v. absorption (abs) of the filtrate at 285 nm to determine the abs(sample). Compare against known vitamin E acetate standard (std).

Procedure for Preparing Standard Solution

1. Weigh 150 mg of vitamin E acetate oil standard in a suitable container.
2. Add 86.0 g of light mineral oil.
3. Mix using a mechanical shaker at medium speed for 15 minutes.
4. Remove 2.5 g of resulting solution and transfer to an appropriate container.
5. Add 43.0 g of light mineral oil to the solution in the container and shake for an additional 15 minutes.
6. Measure the u.v. absorption of above solution at 285 nm to determine abs (std).

Calculations:

$$\frac{X(g)/43.0}{0.150/(0.150+86.0) \times 2.5/(2.5 + 43.0)} = \frac{X(g)}{0.004114}$$

$$X(g) = \frac{abs(sample)}{abs(std)} \times 0.004114$$

% Free oil in powder (% unencapsulated vitamin E acetate) = X/(0.25 x 0.75) x 100

Procedure for Evaluating Powder Suitability for Tabletting

The powders of the present invention are useful for producing antioxidant tablets. To demonstrate their suitability and tabletting performance in antioxidant tablets, tablets were prepared at 1815 kg (4,000 lb) pressure using a rotary press.

| TABLETTING PERFORMANCE OF VITAMIN E ACETATE 75% POWDERS IN ANTIOXIDANT TABLETS AT 1815 KG (4000 LB) PRESSURE USING ROTARY PRESS | | | | | | |
|---|---|---|---|---|---|---|
| Ejection Force | kg (lbs) | 21.3 (47) | 18.1 (40) | 18.6 (41) | 17.2 (38) | 19.5 (43) |
| Hardness, avg. | scu[4] | 15 | 17.1 | 16.9 | 12.3 | 12.9 |
| Hardness, range | | 11.7-16.9 | 15.6-18.3 | 15.8-18 | 11.1-13.6 | 11.8-13.6 |
| Disintegration | minutes | 15 | 15 | 20 | 14 | 14 |
| Friability | % | 0.03 | 0.07 | 0.09 | 0.08 | 0.09 |

[4]scu denotes Strong-Cobb units, as measured by the Strong-Cobb tablet hardness tester (see for example The Theory and Practice of Industrial Pharmacy, edited by Leon Lachman, Herbert A. Lieberman and Joseph L. Kanig, 3rd Edn., Lea & Febiger, Philadelphia 1986, pages 297-299, and Remington's Pharmaceutical Sciences, 18th Edn., Mack Publishing Company, Pennsylvania, page 1639).

The above results were obtained using the following antioxidant formulation:

| ANTIOXIDANT FORMULATION USING VITAMIN E ACETATE 75%, BETATAB R 7.5% AND VITAMIN C-90 GLOBAL | | | | | |
|---|---|---|---|---|---|
| Ingredients | claimed per tablet | % overage | mg/tablet | % | kg/90kg Batch |
| BetaTab R 7.5% (Beta Carotene 7.5% Beadlets) | 6 mg | 35 | 108 | 11.55 | 10.395 |
| Vitamin C-90 Global | 250 mg | 5 | 292 | 31.23 | 28.107 |
| Vitamin E acetate 75% | 200 IU | 5 | 280 | 29.95 | 26.955 |
| Micro-Cel C[5] (Calcium silicate) | | | 97 | 10.37 | 9.333 |
| Avicel® PH 102 (Microcrystalline cellulose) | | | 56 | 5.99 | 5.373 |
| Polyplasdone XL[6] (Crospovidone) | | | 97 | 10.37 | 9.333 |
| Cab-O-Sil® (Colloidal silicon dioxide) | | | 5 | 0.54 | 0.486 |
| TOTAL | | | 935 | 100 | 90 |

[5]A grade of synthetic calcium silicate manufactured by Manville Filtration & Minerals,
[6]Crosslinked, insoluble homopolymer of N-vinyl-2-pyrrolidone in finely divided pulverous form (white), manufactured by GAF Co., N.J., USA.

EXAMPLE 4

High Potency Vitamin E Acetate Formulation Containing Gum Acacia

| Ingredients | 75% E | 80% E |
| --- | --- | --- |
| 1. Vitamin E acetate | 75.0* | 80.0* |
| 2. Methocel® E15LV | 15.0 | 12.0 |
| 3. Gum acacia | 10.0 | 8.0 |
| Total | 100.0% | 100.0% |

*Additional 2% overage was added.

PROCEDURE

1. Aqueous gum acacia solution (20% by weight) was prepared in a 4 l beaker as follows:

| Preparation of 20% gum acacia solution | | | | |
| --- | --- | --- | --- | --- |
| Time (min.) | Temp. (°C) | Stirrer | Amount | Comments |
| 0 | 21 °C | on | 2 l | Add water into beaker |
| 151 | 39 °C | on, slow | 0.5 kg | Add gum acacia |
| 175 | 36 °C | on, fast | | Addition completed |
| 190 | 37 °C | on, fast | | Begin heating |
| 208 | 63 °C | on, slow | | |
| 279 | 71 °C | - - | | Add to emulsion |

2. 10% Aqueous Methocel® E 15LV solution was prepared as in Example 1.

3. Vitamin E acetate was added to the Methocel® solution according to the above formulations and the mixture homogenized to produce an emulsion as in Example 1.

4. The resulting emulsion was heated to 80-90°C with slow (200 rpm) agitation.

5. An appropriate amount of 20% gum acacia solution from step 1 was added.

6. The resulting mixture was blended with gentle agitation for an additional 15 minutes while the temperature was maintained at 70-90°C.

7. Spray dry the vitamin E acetate suspensions under the following conditions.

| Spray drying of the vitamin E acetate suspension | | | | | | | |
|---|---|---|---|---|---|---|---|
| The suspension was pumped from the FRYMA processing unit to the spray tower by a gear pump and then atomized by a rotary atomizer, model SH 36-150/F 15, manufactured by Niro, Inc. | | | | | | | |
| Time (min.) | Set inlet temp. (°C) | Inlet temp. (°C) | Set outlet temp. (°C) | Outlet temp. (°C) | Set pressuret tower in mbar | Pressure tower in mbar | Inlet air flow in m³/h | Comments |
| 0 | 180 | | 100 | | -5 | | | Start up lower |
| 50 | 180 | 181 | 100 | 101 | -5 | -3 | 1500 | Start spray |
| 60 | 180 | 181 | 100 | 100 | -5 | -3 | 1500 | |
| 62 | | | | | | | | Spray drying ended |

EXAMPLE 5

30% Vitamin A Products using Hydroxypropyl Methylcellulose as the Primary Polymer, and Sodium Alginate, Hydroxypropyl Methylcellulose Phthalate or Gum Acacia as the Secondary Polymer

| Ingredient | Formulation I | Formulation II | Formulation III |
|---|---|---|---|
| Vitamin A palmitate | 30.0 + 2.5* | 30.0 + 2.5* | 30.0 +2.5* |
| Butylated hydroxyanisole (BHA) | 1.0 | 1.0 | 1.0 |
| Butylated hydroxytoluene (BHT) | 3.0 | 3.0 | 3.0 |
| Methocel® E15LV | 50.8 | 42.4 | 50.8 |
| HPMCP HP-55S | - | 21.1 | - |
| Gum acacia | - | - | 12.7 |
| Sodium alginate | 3.4 | - | - |
| Calcium chloride | 1.7 | - | - |
| PHARMACOAT 606 | 7.6 | - | - |

Note: * % Overage; all the remaining figures are percentages.

PROCEDURE

Formulation I

Composition

| Material | content (%) | weight (kg) |
|---|---|---|
| Vitamin A palmitate 1.7 MIU/g | 32.47 | 1.30 |
| BHA | 1 | 0.04 |
| BHT | 3 | 0.12 |
| METHOCEL® E15LV premium | 50.82 | 2.03 |
| Sodium alginate | 3.39 | 0.136 |
| Calcium chloride | 1.69 | 0.068 |
| PHARMACOAT 606 | 7.62 | 0.305 |

General

All the solutions were prepared using degassed deionized water under nitrogen. Vitamin A palmitate was shielded from light.

Preparation of a 12% aqueous solution of METHOCEL® E15LV

Water (8 l) was heated to 85°C. METHOCEL® E15LV powder (2.03 kg) was slowly added to the water while mixing with rapid agitation until a uniform suspension was obtained, and thoroughly dispersed. Additional cold water (6.89 l) was quickly added to the suspension and the temperature was reduced to 25°C.

Preparation of a 5% aqueous solution of sodium alginate

Sodium alginate powder (0.136 kg) was added to 2.58 liters of cold water with constant agitation, and the mixture heated to 80-90°C. The resulting solution was kept at 70°C until used.

Preparation of a 5% aqueous solution of calcium chloride

Calcium chloride (0.068 kg) was dissolved in 1.29 l of water. The solution was kept at room temperature until used.

Preparation of a 12% aqueous solution of PHARMACOAT 606

Water (0.75 l) was heated to 90°C. PHARMACOAT 606 powder (0.305 kg) was slowly added to the water while mixing with constant agitation until a uniform suspension was formed. Cold water (1.50 l) was added quickly to the suspension and the temperature was reduced to 25°C. The solution was maintained at room temperature with gentle agitation until used.

Preparation of the emulsion/suspension

BHA (0.04 kg) and 0.12 kg of BHT were added to 1.3 kg of vitamin A palmitate and mixed until a clear solution was formed. The resulting solution was added to the METHOCEL® E15LV solution and emulsified for 30 minutes with the colloidal mill. The oil droplet size measured using a MALVERN Autosizer 2C was 757 nm. The emulsion was then heated to 75°C while slowly mixing (200 rpm) with an anchor stir bar to form a suspension. The sodium alginate solution at 70°C was added to the suspension, and the mixture stirred for 15 minutes. Then the calcium chloride solu-

**14**

tion was added, and the mixture was cooled. The PHARMACOAT solution was added at room temperature and the whole mixed for 10 minutes. The mixture was then heated to 65°C while mixing slowly with an anchor stir bar. The suspension had a viscosity of 11.4 Nsec/m$^2$ (11,400 cP)/65°C. The suspension was then diluted with 2 l of hot water to adjust the viscosity to 2.5 Nsec/m$^2$ (2,500 cP)/65°C.

Spray drying

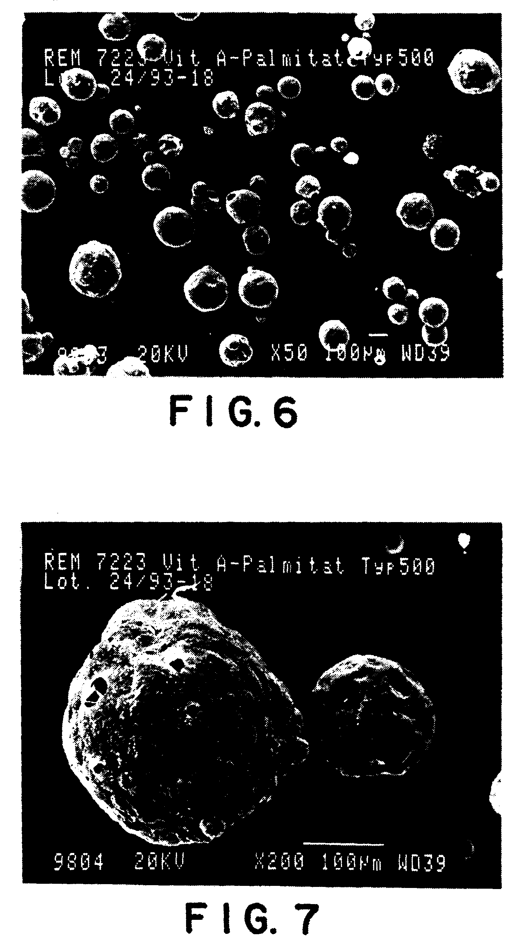

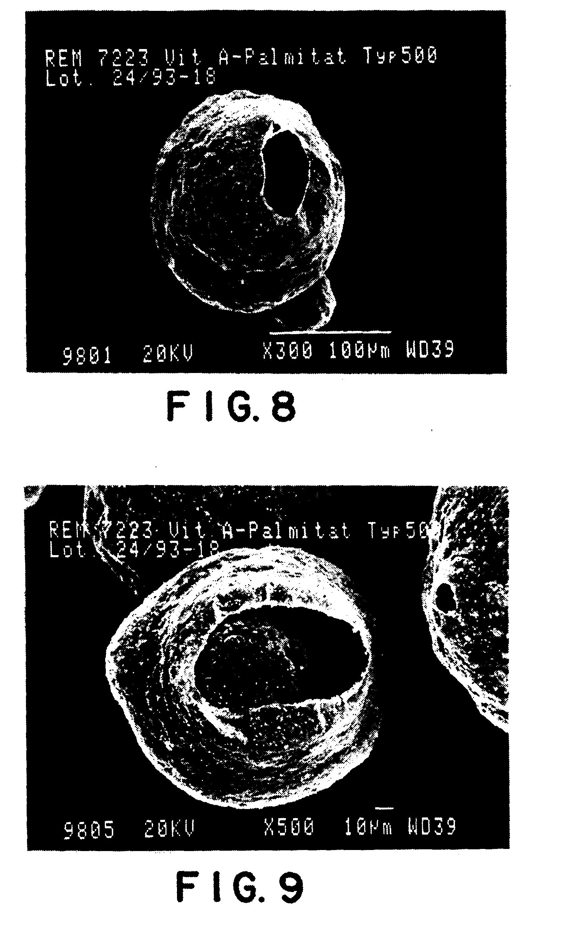

See below (spray dry results are shown in Figures 6-9 given in Example 6 hereinafter).

Formulation II

Composition

| Material | content (%) | weight (kg) |
|---|---|---|
| Vitamin A palmitate 1.7 MIU/g | 32.47 | 1.46 |
| BHA | 1 | 0.045 |
| BHT | 3 | 0.135 |
| METHOCEL® E15LV premium | 42.44 | 1.91 |
| HPMCP HP-55S | 21.09 | 0.95 |

General

All the solutions were prepared using degassed deionized water under nitrogen. Vitamin A palmitate was shielded from light.

Preparation of a 12% aqueous solution of METHOCEL® E15LV

Water (8 l) was heated to 85°C. METHOCEL® E15LV powder (2.03 kg) was slowly added to the water while mixing with rapid agitation until a uniform suspension was obtained, and thoroughly dispersed. Additional cold water (6.89 l) was quickly added to the suspension and the temperature was reduced to 25°C.

Preparation of a 15% aqueous solution of HPMCP

HPMCP powder (0.95 kg) was added to 4.28 l of 0.5 N NaOH with constant agitation, and the mixture heated to 70°C. Hot (70°C) water (1.1 l) was added after the powder had completely dissolved. The solution was kept at 70°C until used.

Preparation of the emulsion/suspension

BHA (0.045 kg) and 0.135 kg of BHT were added to 1.46 kg of vitamin A palmitate and mixed until a clear solution was formed. The solution was added to the METHOCEL® E 15LV solution and emulsified for 30 minutes using a colloidal mill. The oil droplet size measured using a particle size analyzer (MALVERN Autosizer 2C manufactured by Malvern Instruments, Inc.) was 888 nm. Then, the emulsion was heated to 75°C while slowly stirring (200 rpm) with an anchor stir bar to form a suspension. The HPMCP solution at 70°C was added to the suspension and the mixture continuously stirred with an anchor agitator. The pH was adjusted to 6.9 with 0.5 l of 1 N NaOH (pH before: 5.28). The resulting suspension had a viscosity of 0.5 Nsec/m$^2$ (500 cP)/70°C.

Spray drying

See below.

Formulation III

Composition

| Material | content (%) | weight (kg) |
|---|---|---|
| Vitamin A palmitate 1.7 MIU/g | 32.47 | 1.46 |
| BHA | 1 | 0.045 |
| BHT | 3 | 0.135 |
| METHOCEL® E15LV premium | 50.82 | 2.29 |
| Gum acacia | 12.7 | 0.58 |

General

All solutions were prepared using degassed deionized water under nitrogen. Vitamin A palmitate was shielded from light.

Preparation of a 10% aqueous solution of METHOCEL® E 15LV

Water (6.2 l) was heated to 85°C. METHOCEL® E15LV powder (2.29 kg) was slowly added to the water while mixing with rapid agitation until a uniform suspension was obtained. Additional cold water (14.41 l) was quickly added to the suspension and the temperature was reduced to 25°C.

Preparation of a 20% aqueous solution of gum acacia

Gum acacia powder (0.58 kg) was dissolved in 2.32 l of cold water, and the solution heated to 70°C and kept at 70°C until used.

Preparation of the emulsion/suspension

BHA (0.045 kg) and 0.135 kg of BHT were added to 1.46 kg of vitamin A palmitate and mixed until a clear solution was formed. The solution was added to the METHOCEL® E 15LV solution and emulsified for 30 minutes with the colloidal mill. The oil droplet size measured using the MALVERN Autosizer 2C was 854 nm. The emulsion was then heated to 75°C while mixing with an anchor stir bar to form a suspension. The gum acacia solution of 70°C was added to the suspension, and the mixture stirred with an anchor agitator. The pH was adjusted to 7.2 with 40 ml of 0.5 N NaOH (pH before: 6.37). The resulting suspension had a measured viscosity of 1.06 Nsec/m$^2$ (1060 cP)/70°C.

Spray drying

See below.

| Spray Drying Conditions for Formulations I, II and III | | | | | | | |
|---|---|---|---|---|---|---|---|
| Formulation | Feed temp. (°C) | Viscosity in Nsec/m$^2$ (cP equivalent in parenthesis) | pH | Inlet air temp. (°C) | Outlet air temp. (°C) | Inlet air flow m$^3$/h | Wheel speed (rpm) |
| I | 65 | 2.5 Nsec/m$^2$ (2,500)/65°C | -- | 165 | 100 | 1,500 | 8,500 |

(continued)

| Spray Drying Conditions for Formulations I, II and III | | | | | | | |
|---|---|---|---|---|---|---|---|
| Formulation | Feed temp. (°C) | Viscosity in Nsec/m$^2$ (cP equivalent in parenthesis) | pH | Inlet air temp. (°C) | Outlet air temp. (°C) | Inlet air flow m$^3$/h | Wheel speed (rpm) |
| II | 70 | 0.5 Nsec/m$^2$ (500)/70°C | 6.9 | 165 | 100 | 1,500 | 8,500 |
| III | 70 | 1.06 Nsec/m$^2$ (1,060)/70°C | 7.2 | 160 | 100 | 1,500 | 8,500 |

2% silicic acid (type FK 320 DS) was added in each trial. The pressure inside the tower was always about - 4 mbar.

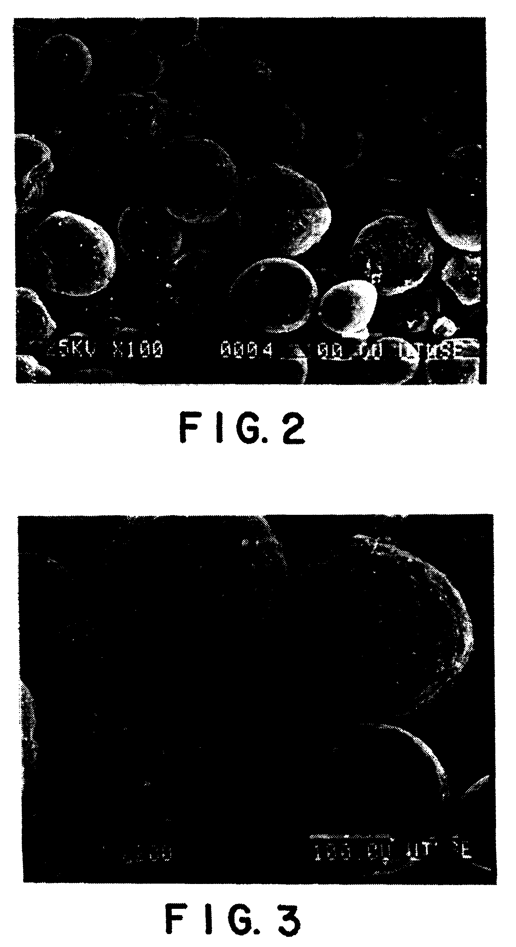

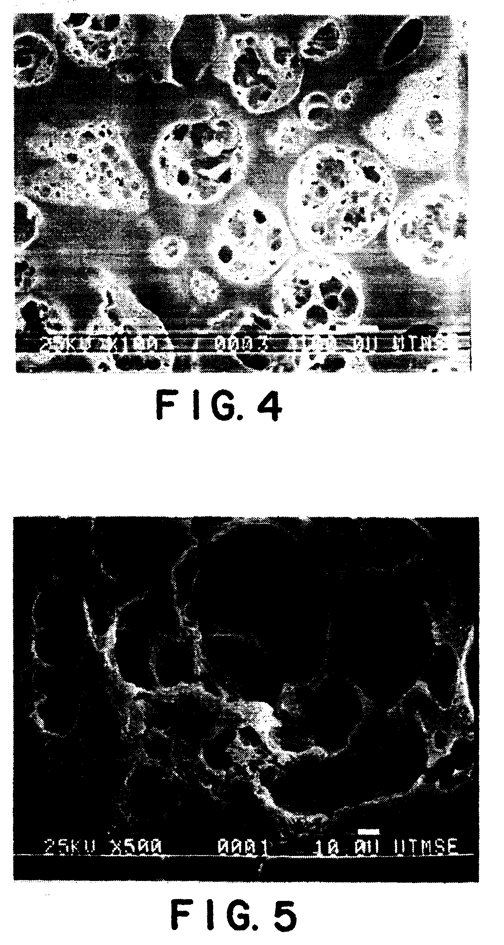

The following Figures illustrate compositions of the present invention, whereby the scanning electron micrograph (SEM) representations (Figures 2-9) resulted from the magnification given in parenthesis [X = times (magnification)].

Brief Description of the Figures

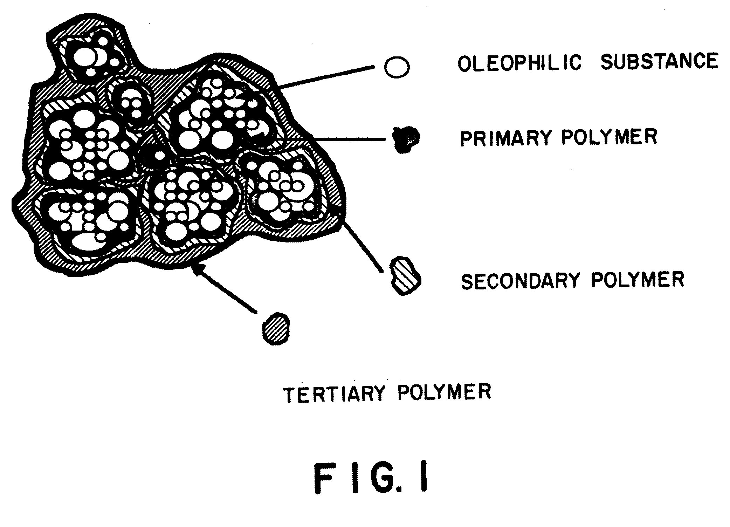

Figure 1 -    An illustration of a powder/beadlet showing the multi-microencapsulation structure formed in one embodiment of the present invention.

Figure 2 -    A scanning electron micrograph (SEM) of a vitamin E powder (100X).

Figure 3 -    SEM of a vitamin E powder (200X).

Figure 4 -    SEM of sectioned vitamin E powder (100X).

Figure 5 -    SEM of sectioned vitamin E powder (500X).

Figure 6 -    SEM of vitamin A palmitate (50X).

Figure 7 -    SEM of vitamin A palmitate (200X).

Figure 8 -    SEM of sectioned vitamin A palmitate (300X).

Figure 9 -    SEM of sectioned vitamin A palmitate (500X).

**Claims**

1.   A method for producing a composition of an oleophilic substance encapsulated by a polymer, characterized by

(a) incorporating an oleophilic substance into a primary polymer-containing solution and

(b) solidifying the primary polymer under mixing conditions to encapsulate the oleophilic substance in the polymer and thereby form the composition of the oleophilic substance encapsulated by the primary polymer, and optionally

(c) mixing the so-produced primary polymer-encapsulated oleophilic substance as particles with a further (secondary) polymer to encapsulate the primary polymer-encapsulated oleophilic substance particles in the secondary polymer and thereby form the composition of the oleophilic substance encapsulated by primary and secondary polymers.

2. A method according to claim 1, wherein step (c) is followed by (d) mixing the so-produced primary and secondary polymer-encapsulated oleophilic substance as particles with a further (tertiary) polymer to encapsulate the primary and secondary polymer-encapsulated oleophilic substance particles in the tertiary polymer and thereby form the composition of the oleophilic substance encapsulated by primary, secondary and tertiary polymers.

3. A method according to claim 1 or 2, wherein the oleophilic substance is vitamin A, D, E or K or a derivative thereof.

4. A method according to claim 3, wherein the derivative is vitamin A acetate, vitamin A palmitate or vitamin E acetate.

5. A method according to any preceding claim, wherein the primary polymer is a cellulose, preferably methyl cellulose or hydroxypropyl methylcellulose.

6. A method according to any preceding claim, wherein the secondary and/or tertiary polymer, if utilized, is a cellulose, preferably methyl cellulose or hydroxypropyl methylcellulose; a cellulose derivative, preferably hydroxypropyl methylcellulose phthalate; a maltodextrin, preferably maltodextrin having a dextrose equivalent value of about 18; an alginic acid derivative, preferably sodium, potassium or propylene glycol alginate; calcium lactate; gum acacia; a gelatin, preferably fish gelatin; or modified starch, such as hydroxypropyl starch or pregelatinized corn starch.

7. A method according to any preceding claim, wherein the step (a) is effected by (1) emulsifying an oleophilic vitamin in an aqueous solution of the primary polymer, or (2) dispersing an oleophilic vitamin in the form of crystals in an aqueous solution of the primary polymer to form a suspension, or (3) dissolving and/or diluting a fat-soluble vitamin in an oil and then emulsifying the vitamin-oil solution in an aqueous solution of the primary polymer to form an emulsion.

8. A method according to any preceding claim, wherein an emulsion or suspension of the oleophilic substance in the primary polymer-containing solution from step (a) is in step (b) either heated to a polymeric setting temperature under mixing conditions, or cross-linked.

9. A method according to any preceding claim, wherein step (c) and/or step (d), if utilized, is achieved by changing the pH, by adding a cross-linking agent, by heating or by spray drying.

10. A method according to any preceding claim, wherein the concentration of the oleophilic substance in the composition ranges from 30% to 90% based on the dry weight of the final encapsulated composition.

**Patentansprüche**

1. Verfahren zur Herstellung einer Zusammensetzung einer mit einem Polymer eingekapselten oleophilen Substanz, gekennzeichnet durch

   (a) Einarbeiten einer oleophilen Substanz in eine ein primäres Polymer enthaltende Lösung und
   (b) Verfestigen des primären Polymers unter Mischbedingungen, um die oleophile Substanz in dem Polymer einzukapsein und dadurch die Zusammensetzung der mit dem primären Polymer eingekapselten oleophilen Substanz zu bilden und gegebenenfalls
   (c) Vermischen der so hergestellten mit primären Polymer eingekapselten, oleophilen Substanz als Teilchen in einem weiteren (sekundären) Polymer, um die mit primärem Polymer eingekapselten oleophilen Substanzteilchen in dem sekundären Polymer einzukapseln und dadurch die Zusammensetzung der mit primären und sekundären Polymer eingekapselten oleophilen Substanz zu bilden.

2. Verfahren nach Anspruch 1, wobei Schritt (c) von Schritt (d) nämlich Vermischen der so hergestellten mit primären oder sekundären Polymer eingekapselten oleophilen Substanz als Teilchen mit einem weiteren (tertiären) Polymer, um die mit primären und sekundären Polymer eingekapselten oleophilen Substanzteilchen in dem tertiären Polymer einzukapseln und dadurch die Zusammensetzung der mit primären, sekundärem und tertiärem Polymer eingekapselten oleophilen Substanz zu bilden, gefolgt wird.

3. Verfahren nach Anspruch 1 oder 2, wobei die oleophile Substanz Vitamin A, D, E oder K oder ein Derivat davon ist.

4. Verfahren nach Anspruch 3, wobei das Derivat ein Vitamin-A-Acetat, Vitamin-A-Palmitat oder Vitamin-E-Acetat ist.

**5.** Verfahren nach einem vorangehenden Anspruch, wobei das primäre Polymer eine Cellulose, vorzugsweise Methylcellulose oder Hydroxypropylmethylcellulose, ist.

**6.** Verfahren nach einem vorangehenden Anspruch, wobei das sekundäre und/oder tertiäre Polymer, falls angewendet, eine Cellulose, vorzugsweise Methylcellulose oder Hydroxypropylmethylcellulose; ein Cellulosederivat, vorzugsweise Hydroxypropylmethylcellulosephthalat; ein Maltodextrin, vorzugsweise Maltodextrin mit einem Dextroseäquivalentwert von etwa 18; ein Alginsäurederivat, vorzugsweise Natrium-, Kalium- oder Propylenglycolalginat; Calciumlactat; Akaziengummi; eine Gelatine, vorzugsweise Fischgelatine; oder modifizierte Stärke, wie Hydroxypropylstärke oder vorgelierte Maisstärke, ist.

**7.** Verfahren nach einem vorangehenden Anspruch, wobei der Schritt (a) durch (1) Emulgieren eines oleophilen Vitamins in einer wässerigen Lösung des primären Polymers, oder (2) Dispergieren eines oleophilen Vitamins in Form von Kristallen in einer wässerigen Lösung des primären Polymers unter Bildung einer Suspension, oder (3) Auflösen und/oder Verdünnen eines fettlöslichen Vitamins in einem Öl und anschließend Emulgieren der Vitamin-Öl-Lösung in einer wässerigen Lösung des primären Polymers, unter Bildung einer Emulsion bewirkt wird.

**8.** Verfahren nach einem vorangehenden Anspruch, wobei eine Emulsion oder Suspension der oleophilen Substanz in der das primäre Polymer enthaltenden Lösung von Schritt (a) in Schritt (b) entweder unter Mischbedingungen auf eine Härtungstemperatur des Polymers erhitzt, oder vernetzt wird.

**9.** Verfahren nach einem vorangehenden Anspruch, wobei Schritt (c) und/oder Schritt (d), falls angewendet, durch Ändern des pH-Werts, durch Zugabe eines Vernetzungsmittels, durch Erhitzen oder durch Sprühtrocknen erreicht werden.

**10.** Verfahren nach einem vorangehenden Anspruch, wobei die Konzentration der oleophilen Substanz in der Zusammensetzung, bezogen auf das Trockengewicht der eingekapselten fertigen Zusammensetzung, im Bereich von 30% bis 90% liegt.

**Revendications**

**1.** Procédé de production d'une composition d'une substance oléophile encapsulée par un polymère, caractérisé par

(a) l'incorporation d'une substance oléophile dans une solution contenant un polymère primaire, et
(b) la solidification du polymère primaire dans les conditions d'un mélange, pour encapsuler la substance oléophile dans le polymère et de ce fait former la composition de la substance oléophile encapsulée par le polymère primaire, et éventuellement
(c) le mélange de la substance oléophile ainsi produite, sous forme de particules, encapsulée par le polymère primaire, à un autre polymère (un polymère secondaire) pour encapsuler dans le polymère secondaire les particules de la substance oléophile encapsulée par le polymère primaire et de ce fait former la composition de la substance oléophile encapsulée par le polymère primaire et le polymère secondaire.

**2.** Procédé selon la revendication 1, dans lequel l'étape (c) est suivie (d) du mélange de la substance oléophile ainsi produite, sous forme de particules, encapsulée par le polymère primaire et le polymère secondaire, à un autre polymère (un polymère tertiaire) pour encapsuler dans le polymère tertiaire les particules de la substance oléophile encapsulée par le polymère primaire et le polymère secondaire et de ce fait former la composition de la substance oléophile encapsulée par le polymère primaire, le polymère secondaire et le polymère tertiaire.

**3.** Procédé selon la revendication 1 ou 2, dans lequel la substance oléophile est la vitamine A, D, E ou K, ou un de leurs dérivés.

**4.** Procédé selon la revendication 3, dans lequel le dérivé est l'acétate de vitamine A, le palmitate de vitamine A ou l'acétate de vitamine E.

**5.** Procédé selon l'une quelconque des revendications précédentes, dans lequel le polymère primaire est une cellulose, de préférence la méthylcellulose ou l'hydroxypropylcellulose.

**6.** Procédé selon l'une quelconque des revendications précédentes, dans lequel le polymère secondaire et/ou le polymère tertiaire, s'ils sont utilisés, sont chacun une cellulose, de préférence, la méthylcellulose ou l'hydroxyméthylpropylcellulose; un dérivé de la cellulose, de préférence le phtalate d'hydroxypropylméthylcellulose; une maltodextrine, de préférence une maltodextrine ayant un équivalent de dextrose d'environ 18 ; un dérivé de l'acide alginique, de préférence l'alginate de sodium, de potassium ou de propylèneglycol ; le lactate de calcium ; la gomme arabique ; une gélatine, de préférence la gélatine de poisson ; ou un amidon modifié, tel que l'hydroxypropylamidon ou l'amidon de maïs prégélatinisé.

7. Procédé selon l'une quelconque des revendications précédentes, dans lequel l'étape (a) est mise en oeuvre par (1) émulsification d'une vitamine oléophile dans une solution aqueuse du polymère primaire, ou (2) dispersion d'une vitamine oléophile sous forme de cristaux dans une solution aqueuse du polymère primaire pour former une suspension, ou (3) dissolution et/ou dilution, dans une huile, d'une vitamine soluble dans les graisses, puis émulsification de la solution huileuse de la vitamine dans une solution aqueuse du polymère primaire pour former une émulsion.

8. Procédé selon l'une quelconque des revendications précédentes, dans lequel une émulsion ou une suspension de la substance oléophile se trouvant dans la solution contenant le polymère primaire obtenue dans l'étape (a) est, dans l'étape (b), soit chauffée à une température de durcissement du polymère dans les conditions d'un mélange, soit réticulée.

9. Procédé selon l'une quelconque des revendications précédentes, dans lequel l'étape (c) et/ou l'étape (d), si elles sont utilisées, sont mises en oeuvre par changement du pH, par addition d'un agent de réticulation, par chauffage ou par séchage par atomisation.

10. Procédé selon l'une quelconque des revendications précédentes, dans lequel la concentration de la substance oléophile dans la composition est comprise entre 30 et 90 % par rapport au poids sec de la composition encapsulée finale.

OLEOPHILIC SUBSTANCE

PRIMARY POLYMER

SECONDARY POLYMER

TERTIARY POLYMER

# FIG. 1

EP 0 768 870 B1

F I G. 2

F I G. 3

FIG. 4

FIG. 5

F I G. 6

F I G. 7

**F I G. 8**

**F I G. 9**